# EUROPEAN PATENT SPECIFICATION

(11) **EP 1 701 699 B1**
(45) Date of publication and mention of the grant of the patent: **13.07.2011**
(21) Application number: 04814759.9
(22) Date of filing: 17.12.2004
(51) Int. Cl.: A61K 9/00, A61K 9/48, A61K 31/505

(54) **Pharmaceutical compositions of an A2a receptor antagonist**
Pharmazeutische Zusammensetzungen eines A2a Rezeptorantagonisten
Compositions pharmaceutiques d'un antagoniste de recepteur A2a

(30) Priority: 19.12.2003 US 531561 P
(43) Date of publication of application: 20.09.2006
(73) Proprietor: Schering Corporation, Kenilworth, NJ 07033-0530 (US)
(72) Inventor: HEIMBECHER, Susan, K., Morris Plains, New Jersey 07950 (US); KOU, Jim, H., Basking Ridge, New Jersey 07920 (US); KAZAKEVICH, Irina, Rockaway, New Jersey 07866 (US)
(74) Representative: Buchan, Gavin MacNicol
(86) International application number: PCT/US2004/042616
(87) International publication number: WO 2005/060981

(56) References cited:
- WO-A-00/10391
- WO-A-00/15231
- WO-A-01/92264
- BARALDI, PIER GIOVANNI ET AL: "7-Substituted 5-Amino-2-(2-furyl)pyrazolo[4,3-e]-1,2,4-t riazolo[1,5- c]pyrimidines as A2A Adenosine Receptor Antagonists: A Study on the Importance of Modifications at the Side Chain on the Activity and Solubility" JOURNAL OF MEDICINAL CHEMISTRY , 45(1), 115-126 CODEN: JMCMAR; ISSN: 0022-2623, 2002, XP008055018

## Description

The present invention relates to formulations containing a compound of Formula 1 and at least one anionic surfactant that forms an ion pair with the compound of Formula 1, wherein the anionic surfactant is sodium docusate. These formulations are useful in the treatment of, *inter alia*, Parkinson's disease.

Adenosine is known to be an endogenous modulator of a number of physiological functions. At the cardiovascular system level, adenosine is a strong vasodilator and a cardiac depressor. On the central nervous system, adenosine induces sedative, anxiolytic and antiepileptic effects. On the respiratory system, adenosine induces bronchoconstriction. At the kidney level, it exerts a biphasic action, inducing vasoconstriction at low concentrations and vasodilation at high doses. Adenosine acts as a lipolysis inhibitor on fat cells and as an antiaggregant on platelets.

Adenosine action is mediated by the interaction with different membrane specific receptors which belong to the family of receptors coupled with G proteins. Biochemical and pharmacological studies, together with advances in molecular biology, have allowed the identification of at least four subtypes of adenosine receptors: A₁, A₂ₐ, A_{2b} and A₃. A₁ and A₃ are high-affinity, inhibiting the activity of the enzyme adenylate cyclase, and A₂ₐ and A_{2b} are low-affinity, stimulating the activity of the same enzyme. Analogs of adenosine able to interact as antagonists with the A₁, A₂ₐ, A_{2b} and A₃ receptors have also been identified.

Selective antagonists for the A₂ₐ receptor are of pharmacological interest because of their reduced level of side affects. In the central nervous system, A₂ₐ antagonists can have antidepressant properties and stimulate cognitive functions. Moreover, data has shown that A₂ₐ receptors are present in high density in the basal ganglia, known to be important in the control of movement. Hence, A₂ₐ antagonists can improve motor impairment due to neurodegenerative diseases such as Parkinson's disease, senile dementia as in Alzheimer's disease, and psychoses of organic origin.

US 2002/0099061 discloses novel compounds that are A₂ₐ receptor antagonists. One particular compound, 7-[2-[4-[4-(methoxyethoxy)phenyl]1-piperazinyl]ethyl]-2-(2-furanyl)-7H-pyrazolo[4,3-e]triazolo[1,5-c]pyrimidin-5-amine, has shown considerable potency as an A₂ₐ receptor antagonist. As such, it would be useful to have a variety of pharmaceutical formulations that contain this compound. More particularly, it would be useful to have stabilized liquid formulations containing this compound and other compounds of this nature and/or stabilized solid dosage forms of this or similar compounds which provide adequate dissolution. It would also be useful to optimize the absorption profile of this compound or similar compounds of this nature.

The aqueous solubility of any pharmaceutically active ingredient is recognized as a critical parameter in both liquid and solid dosage forms. For liquid formulations in particular, the solubility of the drug substance in water will limit the concentration that can be achieved in the aqueous formulation. Similarly for solids, both the rate and the extent of dissolution can be affected by the aqueous solubility of the active ingredient. This in turn can affect the rate and extent of absorption of the active ingredient from the gastrointestinal tract. For non-aqueous formulations, both dosage form selection and concentration can be limited by the non-aqueous solubility of a drug substance, e.g., in an emulsion, lipid and/or cosolvent based formulations. Non-aqueous formulations may generally be referred to as either lipid based formulations which may consist of pure oil or oils or as co-solvent based formulations which may consist of water soluble organic materials such as ethanol, propylene glycol and polyethylene glycols (PEG). Ionic surfactants such as sodium lauryl sulfate, sodium dodecyl sulfate and docusate sodium and non-ionic surfactants such as poloxamers (pluronic) and polysorbates (Tweens) may also be included. Additionally, lipid formulations make up the hydrophobic phase of emulsions, microemulsions and self emulsifying systems. Improvement in the solubility in non-aqueous systems can also lead to improved oral absorption.

There are certain active ingredients that have poor solubility in either aqueous or non-aqueous based formulations or, as in the case of A₂ₐ receptor antagonists described above or other certain active ingredients, may have poor solubility in both aqueous and non-aqueous based formulations. Poor aqueous solubility limits potential, viable formulations and may lead to poor dissolution and /or precipitation and low and /or variable oral absorption. Poor non-aqueous solubility also limits potential, viable formulations and may lead to low and /or variable oral absorption. Accordingly, there is a need for formulations which can provide either improved aqueous or non-aqueous solubility for compounds such as the A₂ₐ receptor antagonist compounds described above, among others, which have neither aqueous nor non-aqueous solubility. It is most preferable to provide formulations which can provide both improved aqueous and non-aqueous solubility for compounds such as the A₂ₐ receptor antagonist compounds described above, among others, in order to allow for the widest selection of liquid or solid formulations and/or optimize dissolution and improve the absorption profile.

### SUMMARY OF THE INVENTION

According to the invention there is provided a pharmaceutically acceptable composition comprising:
a) a compound having the structure according to Formula I 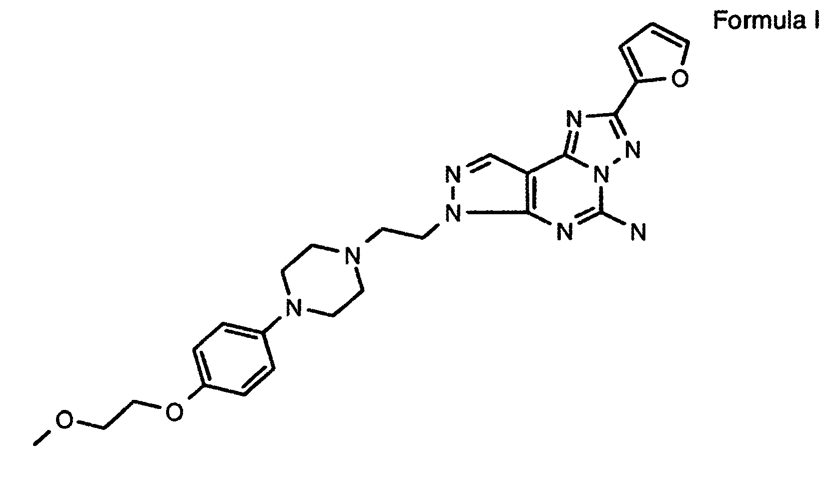 wherein the substituent shown as "N" represents NH₂;
b) at least one pharmaceutically acceptable non-aqueous liquid carrier, wherein the liquid carrier is miscible with an aqueous carrier;
c) at least one acidifying agent; and
d) at least one anionic surfactant, wherein said at least one anionic surfactant forms
   an ion pair with the compound of Formula I and is sodium docusate.

### DETAILED DESCRIPTION OF THE INVENTION

The compound 7-[2-[4-[4-(methoxyethoxy)phenyl]1-piperazinyl]ethyl]-2-(2-furanyl)-7H-pyrazolo[4,3-e]triazolo[1,5-c]pyrimidin-5-amine has the following chemical structure: 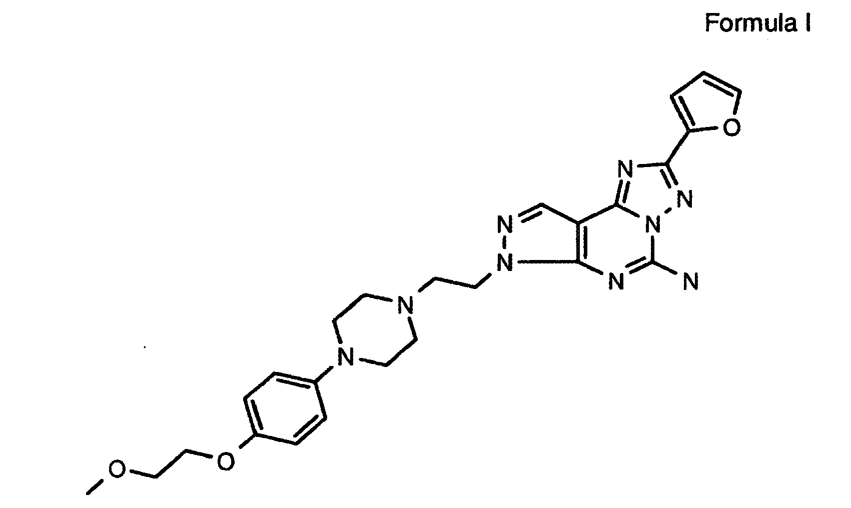 wherein the substituent shown as "N" represents NH₂.

This compound is useful for treating central nervous system diseases such as depression, cognitive diseases and neurodegenerative diseases such as Parkinson's disease, senile dementia or psychoses of organic origin, and stroke in a patient in need of such treatment. In particular, the compound is useful for treating Parkinson's disease. The compound may exist as a free base or as a pharmaceutically acceptable salt. All such acid and base salts are intended to be pharmaceutically acceptable salts within the scope of the invention and all acid and base salts are considered equivalent to the free forms of the corresponding compounds for purposes of the invention.

This compound is virtually insoluble in aqueous media at neutral pH. However, the compound is a weak base with three pKa's of 1.72 (calculated), 2.25 (calculated) and 6.87 (titrated) and it therefore becomes increasingly positively charged as the pH is decreased. As expected, solubility improves with ionization. However, even when essentially all of the compound carries a +1 charge, the aqueous solubility remains poor, e.g., at pH 4 the solubility is < 2 µg/mL. Similarly, at pH 2 the average charge will be +2 but, the solubility for this strongly charged compound is only 1 mg/mL. Based on the pKa values, under still more acidic conditions this compound will have an average charge of +3 and the solubility might be expected to increase further. However, no further increase in solubility has been achieved, i.e., at pH 1.0 (0.1 N HCL) the solubility of this compound decreases to < 1 mg/mL. This type of decrease in solubility is consistent with the formation of insoluble chloride salts.

Typically, pharmaceutical compounds which exhibit little or no aqueous solubility will however be soluble in non-aqueous media. However, as shown in the Table 1 below, the A₂ₐ receptor antagonist compound described above has both low aqueous and non-aqueous solubility.

**Table 1: Formula 1 Aqueous and Non-Aqueous Solubility**

| **Aqueous Solubility** | | **Non-Aqueous Solubility** | |
|---|---|---|---|
| **Diluent** | **Solubility** | **Diluent** | **Solubility** |
| Buffer pH 7.4 | < 5 ng/mL | Ethanol | 0.1 mg/mL |
| Buffer pH 4 | 1.7 µg/mL | Propylene Glycol | 0.03 mg/mL) |
| Buffer pH 3 | 105 µg/mL | Soybean Oil | < 0.07 mg/mL |

Attempts to formulate this compound with certain negatively charged or anionic surfactants, such as sodium lauryl sulfate (SLS) in aqueous media, resulted in ion pairing and a significant decrease in the aqueous solubility of the compound. Surprisingly, however, it was found that the when 7-[2-[4-[4-(methoxyethoxy)phenyl]1-piperazinyl]ethyl]-2-(2-furanyl)-7H-pyrazolo[4,3-e]triazolo[1,5-c]pyrimidin-5-amine was formulated with docusate sodium, in acidified propylene glycol, there was obtained a solution of the active compound at a concentration 80 times greater than when only the active compound and propylene glycol were combined. The solution is acidified in order to ionize the active compound. Docusate sodium is also known as Dioctyl Sodium Sulfosuccinate. Dioctyl Sodium Sulfosuccinate is the sodium salt of the diester of 2-ethylhexyl alcohol and sulfosuccinic acid. It functions as an anionic surfactant.

The anionic surfactant useful in the practice of the present invention contains a negatively charged sulfate group and is Sulfobutanedioc acid bis [2-ethyl-hexyl ester] dioctyl sulfosuccinate (Docusate sodium).

The anionic surfactant is present in the composition of the present invention at levels of 0.005 to 10%, preferably 0.01 to 5.0% by weight in liquid formulations or for solid formulations, it may be present in 1 to 20 times the active compound concentration on a mole to mole basis.

For aqueous formulations of the present invention, sodium docusate is typically added to improve aqueous solubility. However, sodium docusate actually decreases the aqueous solubility of 7-[2-[4-[4-(methoxyethoxy)phenyl]1-piperazinyl]ethyl]-2-(2-furanyl)-7H-pyrazolo[4,3-e]triazolo[1,5-c]pyrimidin-5-amine under acidic conditions. As described above, at pH 2.0 a 0.1 mg/mL solution of 7-[2-[4-[4-(methoxyethoxy)phenyl]1-piperazinyl]ethyl]-2-(2-furanyl)-7H-pyrazolo[4,3-e]triazolo[1,5-c]pyrimidin-5-amine will precipitate out of solution in the presence of sodium docusate. These results indicate that under acidic conditions docusate is not interacting with 7-[2-[4-[4-(methoxyethoxy)phenyl]1-piperazinyl]ethyl]-2-(2-furanyl)-7H-pyrazolo[4,3-e]triazolo[1,5-c]pyrimidin-5-amine as a surfactant, but rather as an ion pair reagent, i.e., at pH 2 docusate and the compound of Formula I will be oppositely charged and form an insoluble, hydrophobic complex. More specifically, by "ion-pair" it is meant to mean the non-covalent bonding of two oppositely charged molecules.

Tween 80, a non-ionic surfactant, amongst others, can be used to improve the aqueous solubility of hydrophobic compounds, and has been found to minimize the precipitation of the compound of Formula I by sodium docusate described above. This effect is assumed to result from solubilization of the compound of Formula I:docusate complex and is consistent with the proposed formation of a hydrophobic ion pair.

Non-ionic surfactant refers to a surfactant which lacks a net ionic charge and does not dissociate to an appreciable extent in aqueous media. The properties of non-ionic surfactants are largely dependent upon the proportions of the hydrophilic and hydrophobic groups in the molecule. Hydrophilic groups include the oxyethylene group (-OCH₂CH₂-) and the hydroxy group. By varying the number of these groups in a hydrophobic molecule, such as an ester of a fatty acid, substances are obtained which range from strongly hydrophobic and water insoluble compounds, such as glyceryl monostearate, to strongly hydrophilic and water-soluble compounds, such as the macrogols. Between these two extremes types include those in which the proportions of the hydrophilic and hydrophobic groups are more evenly balanced, such as the macrogol esters and ethers and sorbitan derivatives. Suitable non-ionic surfactants may be found in Martindale, The Extra Pharmacopoeia, 28th Edition, 1982, The Pharmaceutical Press, London, Great Britain, pp. 370 to 379.

Such suitable non-ionic surfactants include block copolymers of ethylene oxide and propylene oxide, glycol or glyceryl esters of saturated or unsaturated C₈ to C₂₀ acids, preferably, polyoxyethylene esters of saturated or unsaturated C₈ to C₂₀ acids, polyoxyethylene ethers of saturated or unsaturated C₈ to C₂₀ acids, and polyvinylalcohols or sorbitan esters of saturated or unsaturated C₁₀ to C₂₀ acids. Preferably, the non-ionic surfactant is a sorbitan ester of a saturated or unsaturated C₁₀ to C₂₀ acid, and more preferably the sorbitan ester is a fatty acid ester of sorbitan selected from sorbitan monolaurate, sorbitan monooleate, sorbitan sesquioleate, sorbitan trioleate, sorbitan monopalmitate, sorbitan monostearate and sorbitan tristearate, or mixtures thereof.

Suitable sorbitan esters include, e.g. Polysorbate 20, Polysorbate 40, Polysorbate 60, Polysorbate 65, Polysorbate 80, Polysorbate 85, Sorbitan Monolaurate, Sorbitan Mono-oleate, Sorbitan Monopalmitate, Sorbitan Monostearate, Sorbitan Sesquioleate, Sorbitan Trioleate and Sorbitan Tristearate. The most preferred non-ionic surfactant is Polysorbate 80, available from ICI Americas under the tradename Tween 80 which is a mixture of oleate esters of sorbitol and sorbitol anhydrides, consisting predominantly of the monoester, condensed with approximately 20 moles of ethylene oxide.

Suitable block copolymers of ethylene oxide and propylene oxide generically called "Poloxamers" and include those represented by the following chemical structure I: 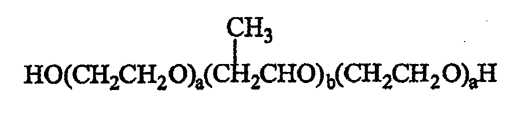 wherein a is an integer ranging from about 10 to about 110, preferably from about 12 to 101; more preferably from about 12 to 80; and
b is an integer ranging from about 20 to about 60, more preferably from about 20 to about 56; also from about 20 to 27.

Suitable glycol and glyceryl esters of fatty acids include glyceryl monooleate and similar water soluble derivatives;

Suitable polyoxyethylene esters of fatty acids (macrogol esters) include polyoxyethylene castor oil and hydrogenated castor oil derivatives;

Suitable polyoxyethylene ethers of fatty acids (macrogol ethers) include Cetomacrogel 1000, Lauromacrogols (a series of lauryl ethers of macrogols of differing chain lengths), e.g. Laureth 4, Laureth 9 and Lauromacrogol 400. Additional non-ionic surfactants may be used to improve the solubility of the ion pair which may be formed between cationic active agents and naturally bile acids *in vivo* to change the absorption profile of the active agent.

The amount of non-ionic surfactant will vary depending on the concentration required to reach the critical micelle concentration (CMC). The non-ionic surfactant can range from concentrations equal or greater than the CMC concentration in liquid formulations. In solid formulations the non-ionic surfactant will be present at concentrations equal or greater to the CMC concentration that will be achieved upon dilution. The expected dilution volume will be the volume required for either reconstitution or a biologically relevant volume such as 250 to 900 mL.

Excess amounts of anionic surfactants can also be used to replace or partially replace non ionic surfactants. In this case, the anionic surfactant is added in an amount equal to that necessary to achieve the CMC. The ion pair formed between the active compound and the anionic surfactant is then solubilized within a micelle that is formed by either the additional anionic surfactant or a mixture of anionic surfactant and nonionic surfactant.

The formulations of the present invention may be administered orally as either a liquid or a solid. The compound of Formula 1 may be present in an amount of about 0.5 to 100 mg, preferably about 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg or 100 mg. Liquid oral formulations may be desirable for improving bioavailability and can include solutions, syrups, elixirs or solutions filled into soft or hard capsules.

Thus, an aqueous/organic oral solution can be prepared using a co-solvent, e.g., an alcohol such as ethanol or a glycol such as polyethylene glycol or propylene glycol and/ or amphiphilic compounds such as mono, di and triglycerides, suitable agents for pH adjustment, e.g., hydrochloric acid or citric acid, and a suitable biocompatible anionic surfactant, i.e., sodium docusate. If greater aqueous solubility is required in order to decrease precipitation of the compound of Formula 1 upon dilution of the formulation with gastric or intestinal fluids, additional amounts of the anionic surfactant and /or a non-ionic surfactant such as Tween-80 can be added. Similarly, oil based oral solutions can be prepared by solubilizing the active principle with a suitable anionic surfactant, i.e., sodium docusate, an acidifying agent and a substantially non-aqueous carrier (excipient). The acidifying agent can be any biocompatible substance that is soluble in the hydrophobic formulation and which provides sufficient acidity for the compound of Formula 1 compound to ionize and form a complex with the anionic surfactant. The non-aqueous carrier can be any substance that is biocompatible and will be in a liquid or will be liquefied in the formulation.

The carrier is usually hydrophobic and commonly organic, e.g., an oil or fat of vegetable, animal, mineral or synthetic origin or derivation. Preferably, but not necessarily, the carrier includes at least one chemical moiety of the kind that typifies "fatty" compounds, e.g., fatty acids, alcohols, esters, etc., i.e., a hydrocarbon chain, an ester linkage, or both. "Fatty" acids in this context include acetic, propionic and butyric acids, through straight- or branched-chain organic acids containing up to 30 or more carbon atoms. Preferably, the carrier is immiscible in water and/or soluble in the substances commonly known as fat solvents. The carrier can correspond to a reaction product of such a "fatty" compound or compounds with a hydroxy compound, e.g., a mono-hydric, di-hydric, trihydric or other polyhydric alcohol, e.g., glycerol, propanediol, lauryl alcohol, polyethylene or -propylene glycol, etc. These compounds include the fat-soluble vitamins, e.g., tocopherols and their esters, e.g., acetates sometimes produced to stabilize tocopherols. Sometimes, for economic reasons, the carrier may preferably comprise a natural, unmodified vegetable oil such as sesame oil, soybean oil, peanut oil, palm oil, or an unmodified fat. Alternatively the vegetable oil or fat may be modified by hydrogenation or other chemical means which is compatible with the present invention. The appropriate use of hydrophobic substances prepared by synthetic means is also envisioned.

As with the aqueous/organic oral solutions, if greater aqueous solubility is required in order to decrease precipitation of the compound of Formula 1 upon dilution of the formulation in vivo, e.g., with gastric or intestinal fluids, additional amounts of the anionic surfactant and /or a non-ionic surfactant can be added. The additional anionic surfactant and/or the non-ionic surfactant in hydrophobic solutions can be any biocompatible surfactants that have sufficient solubility in the substantially non-aqueous carrier. If the anionic and/or non-ionic surfactants are to solubilize the compound of Formula 1 via micelles in aqueous media, they must also have sufficient solubility or dispersibility in aqueous media, e.g., docusate and polyoxyethylene castor oil derivatives (cremophors), respectively.

Solid oral formulations of the compound of Formula 1 can also be prepared using a suitable biocompatible anionic surfactant and excipients so that, upon dilution in vivo with gastric or intestinal fluids, an ion pair between the compound of Formula 1 and the anionic surfactant is formed, i.e. the compound of Formula 1 with sufficient sodium docusate to form an ion pair, i.e., docusate concentration 1 to 20 times the compound of Formula 1 concentration on a mole:mole basis, additional sodium docusate or non-ionic surfactant, i.e., poloxamer 188, to solubilize the compound of Formula 1:anionic surfactant ion pair, required acidifiers, and any fillers, dispersants, flavor agents etc. required for processing and formulating the solid dosage form, i.e., tablets, hard or soft filled capsules.

The formulations of the present invention may also be administered by parenteral routes such as intravenous injection, subcutaneous injection or intramuscular injection in similar amounts. Thus, to prepare an aqueous/organic solution for intravenous injection it is possible to use a co-solvent, e.g., an alcohol such as ethanol or a glycol such as polyethylene glycol or propylene glycol, a suitable agent for pH adjustment, e.g., hydrochloric acid or citric acid and a suitable biocompatible anionic surfactant, e.g., a phospholipid. If greater aqueous solubility is required in order to decrease precipitation upon dilution, i.e., as can occur during dilution in the blood stream or when combined with vehicles for infusion, additional amounts of the anionic surfactant can be added and /or a non-ionic surfactant such as Tween 80 (polysorbate 80) can be added. Oily, injectable solutions, as might be preferred for intramuscular injection, can be similarly prepared by solubilizing the active principle with a suitable anionic surfactant, an acidifying agent and a hydrophobic or non-aqueous carrier. The acidifying agent can be any biocompatible substance which is soluble in the hydrophobic formulation and which provides sufficient acidity for the Formula 1 compound to ionize and form a complex with the anionic surfactant, i.e., sodium docusate.

Another application of this invention is the formation of the compound of Formula 1:anionic surfactant ion pair before addition to the pharmaceutical compositions listed above. In this case, the addition of an acidifying agent or agents to the final pharmaceutical composition would not be required. A procedure for obtaining the compound of Formula 1:anionic surfactant complexes is described in Example 1.

This disclosure also demonstrates solubilization of the compound of Formula 1 through the use of acidified cosolvents without the use of anionic surfactants. This result would not be predicted due to the fact that the uncharged or neutral active compound is more hydrophobic than the ionized compound and would, therefore, be expected to have greater solubility as the percentage of cosolvent is increased. However, the solubility of the neutral compound in propylene glycol is about 0.03 mg/mL, (Table 1), whereas, when the solution is acidified in order to increase the concentration of ionized active compound, the concentration increases to more than 9 mg/mL (Table 2).

**Table 2: Effect of Solution Hydrophobicity on Formula 1 Concentration**

| | **Percent Propylene Glycol In Solution / Dilution Factor (DF)^{b}** | | | | **Soybean Oil** |
|---|---|---|---|---|---|
| **Stock Solutions^{a}** | **86% PG/ (Stock)** | **43% PG /DF2** | **17% PG/ DF4** | **8.6% PG/ DF10** | **Amt. Extracted^{c}** |
| | Formula 1 | Formula 1 | Formula 1 | Formula 1 | Formula 1 |
| 1. Acidified Propylene Glycol | 9.8 mg/mL | 4.6 mg/mL | 1.9 mg/mL | 0.97 mg/mL | 0.006 mg/mL |
| 2. Acidified Propylene | | | | | |
| Glycol + Tween 80 | 8.7 mg/mL | 4.0 mg/mL | 1.8 mg/mL | 0.78 mg/mL | 0.007 mg/mL |
| 3. Acidified Propylene | | | | | |
| Glycol + Docusate sodium | 9.2 mg/mL | 0.14 mg/mL | 0.037 mg/mL | 0.011 mg/mL | 0.28 mg/mL |

| | | | | | |
|---|---|---|---|---|---|
| ^{a}Stock Solutions: 1) Propylene glycol (86 %v/v) + citric acid/ HCl to pH 3.4, 2) Stock Solution 1+ 36 mg/mL Tween 80, 3) Stock Solution 1 + 61 mg/mL docusate sodium. | | | | | |

^{b}Solubilities were determined in the original stock solutions and following 2, 4 and 10x dilutions with deionized water (precipitate remained in all samples upon dilution and supernatant sampled for assay).
^{c}Soluble concentrations of the compound of Formula 1 found in soybean oil following extraction from stock solutions 1, 2 and 3. Excess compound of Formula 1 was added to the stock solutions before extraction.

This phenomenon is consistent with self-association of the active compound (whereby at least one of the molecules in the self-associated complex carries at least one positive charge) via hydrophobic and hydrogen bonding.

In order to determine the effect of solution hydrophobicity on the compound of Formula 1 solubility, the original stock solutions 1, 2 and 3 (Table 2) were made more hydrophilic by diluting them with deionized water. In this way, the concentration of propylene glycol was decreased 10 fold from the initial concentration of 86% to a final concentration of 8.6%. On the other hand, due to the presence of citrate buffer, the pH is not significantly affected by dilution. Similarly, the compound of Formula 1 solubility should not be affected by the 2-10x dilution of sodium docusate as this surfactant will still be present at levels above its critical micelle concentration (CMC). As can be seen in Table 2, the compound of Formula 1 solubility decreases as each of the stock solutions are diluted. However, the decrease in solubility seen with dilution of stock solution #3 is significantly greater than that seen with stock solutions #1 and #2 and indicates that the ion pair between the active compound and docusate is more hydrophobic than the self-associated compound of Formula 1 complex.

Additionally, soybean oil was used to determine the effect of a hydrophobic media on solubility (soybean oil is significantly more hydrophobic than propylene glycol and is non-miscible with water). For this solubility determination the active compound was first added in excess to stock solutions 1-3 and then, each solution was extracted with soybean oil. As can be seen in Table 2, only the solution containing docusate led to significant extraction of the active compound into soybean oil. This result is consistent with the solubilities described above, i.e., the compound of Formula 1:docusate complex is more hydrophobic than either the active molecule alone or the active molecule as a self associated pair and, therefore, should be extracted into a hydrophobic media to the greatest extent (it is assumed that the compound of Formula 1:docusate complex was extracted from stock solution 3, as apposed to just the active molecule).

For preparing pharmaceutical compositions from the compounds described by this invention, inert, pharmaceutically acceptable carriers can be either solid or liquid. Solid form preparations include powders, tablets, dispersible granules, capsules, cachets and suppositories. Examples of pharmaceutically acceptable carriers and methods of manufacture for various compositions may be found in A. Gennaro (ed.), Remington: The Science and Practice of Pharmacy, 20th Edition, (2000), Lippincott Williams & Wilkins, Baltimore, MD.

Liquid form preparations include solutions, suspensions and emulsions. As an example may be mentioned water or water-propylene glycol solutions for parenteral injection or liquid carriers with the addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compositions of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally. Preferably, the pharmaceutical preparation is in a unit dosage form. In such form, the preparations subdivided into suitably sized unit doses containing appropriate quantities of the active component, e.g., an effective amount to achieve the desired purpose.

The actual dosage employed may be varied depending upon the requirements of the patient and the severity of the condition being treated. Determination of the proper dosage regimen for a particular situation is within the skill in the art. For convenience, the total daily dosage may be divided and administered in portions during the day as required.

The amount and frequency of administration of the compound of the invention and/or the pharmaceutically acceptable salts thereof will be regulated according to the judgment of the attending clinician considering such factors as age, condition and size of the patient as well as severity of the symptoms being treated.

There are many benefits to the formulations of the present invention. These benefits include the formation of delivery systems that require non-aqueous solubility, e.g., emulsion, lipid or cosolvent based formulations that can be effectively utilized for 7-[2-[4-[4-(methoxyethoxy)phenyl]1-piperazinyl]ethyl]-2-(2-furanyl)-7H-pyrazolo[4,3-e]triazolo[1,5-c]pyrimidin-5-amine. Additionally, the solubility and dissolution profile for both aqueous and non-aqueous solution formulations can be altered to yield improved bioavailability. Further, the formulations of the present invention can affect salt selection of 7-[2-[4-[4-(methoxyethoxy)phenyl]1-piperazinyl]ethyl]-2-(2-furanyl)-7H-pyrazolo[4,3-e]triazolo[1,5-c]pyrimidin-5-amine which may be extended to include non-aqueous solubility profiles. Finally, the formulations of the present invention may be administered to patients as a non-aqueous or aqueous solution for oral administration, or by intravenous or intramuscular injection and the like.

It will be appreciated by one of skill in the art that this technology may be applied to other forms of delivering pharmaceutically active compounds that have poor aqueous solubility and carry a sufficient positive charge at a neutral pH. For instance, the compounds may be taste masked when used with another component such as an anionic surfactant like Docusate Sodium that is negatively charged at a pH of 7. Taste masking is understood as a perceived reduction of an undesirable taste that would otherwise be there. The mouth is, for the most part, a neutral environment where the pH is about 7. One can mask the unpleasant taste of a positively charged drug that is soluble in the mouth by means of ion pairing it with a negatively charged component to form an insoluble precipitate. This could effectively mask the unpleasant taste of a pharmaceutically active ingredient, i.e., drug or medicine. When taken orally, the pharmaceutically active compound would immediately dissolve in the acidic pH environment in the stomach.

The invention will be further illustrated by the following non-limiting Example.

### Example 1

A non-aqueous formulation of the present invention was prepared as set forth by the following procedure. A first solution was prepared by adding 100 mg of the compound of Formula I above to 500 mL of 0.01 N HCL. Next, a second solution was prepared by adding 20 grams of sodium docusate to 500 mL of 0.01 N HCL. These two solutions were mixed and a complex of the compound of Formula I and docusate sodium was precipitated from solution. The precipitate was thereafter separated by centrifuging the mixture, decanting the supernatant and then drying the residue complex.

The dried residue was then dissolved in a small quantity of propylene glycol and the concentration of the compound of Formula I in this solution was determined to be 2.5 mg/mL. As stated above, the solubility of the compound of Formula I was more than 80 fold greater than the solubility of SCH alone in propylene glycol.

The ability to solubilize the compound of Formula 1 via acidified media either with or without an ion pairing compound allows for greater flexibility in addressing formulation requirements, e.g., for very hydrophobic media the use of ion pair excipients will lead to greater solubility of the compound of Formula 1 whereas, if ion pair reagents are not acceptable for the particular application, significant solubility can still be achieved by utilizing acidified cosolvents alone.

The solubility achieved with solution 2, which contained the non-ionic surfactant Tween 80, was similar to that achieved with solution 1. This further indicates that the solubility effects seen with anionic surfactants such as docusate are due to ion pairing, rather than typical surfactant solubility effects as would be seen by either an ionic or non-ionic surfactants.

The increased solubility in acidified co-solvent solutions is dependent on the pH. As the pH decreases the active compound will become more ionized and therefore more molecules, as well as more sites on the molecule, will be positively charged and available for either self-association and or ion pairing. Therefore, the highest solubility in non-aqueous solutions, will be achieved at lower pH values.

In addition, direct addition to solutions containing acidified propylene glycol and docusate resulted in solutions containing the active compound at a concentration greater than 300 times than the solubility of the active compound in propylene glycol. Similarly, the maximum concentration of the active compound in acidified propylene glycol without docusate was 300 times greater than the solubility of the active compound in propylene glycol.

## Claims

1. A pharmaceutically acceptable composition comprising:
a) a compound having the structure according to Formula I 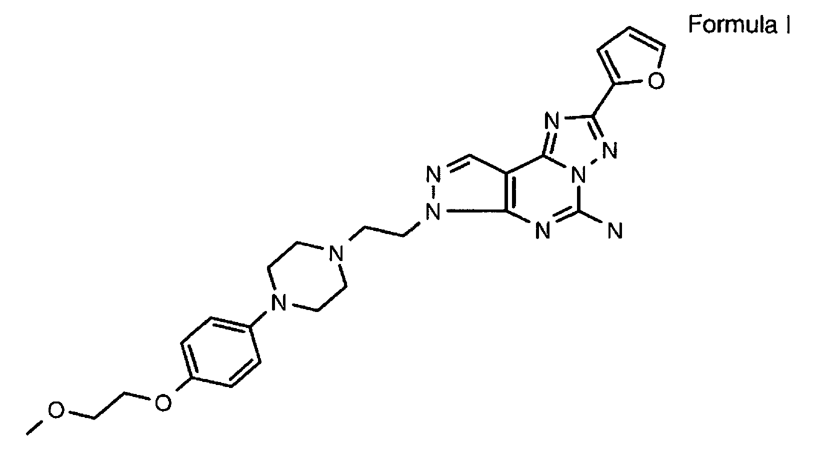 wherein the substituent shown as "N" represents NH₂;
b) at least one pharmaceutically acceptable non-aqueous liquid carrier, wherein the liquid carrier is miscible with an aqueous carrier;
c) at least one acidifying agent; and
d) at least one anionic surfactant, wherein said at least one anionic surfactant forms an ion pair with the compound of Formula I and is sodium docusate.

2. The pharmaceutically acceptable composition according to claim 1, further comprising at least one non-ionic surfactant.

3. The pharmaceutically acceptable composition according to claim 2, wherein the non-ionic surfactant is selected from the group consisting of block copolymers of ethylene oxide and propylene oxide, glycol or glyceryl esters of saturated or unsaturated C₈ to C₂₀ acids, polyoxyethylene esters of saturated or unsaturated C₈ to C₂₀ acids , polyoxyethylene ethers of saturated or unsaturated C₈ to C₂₀ acids, polyvinylalcohols or sorbitan esters of saturated or unsaturated C₁₀ to C₂₀ acids.

4. The pharmaceutically acceptable composition according to claim 2, wherein the at least one non-ionic surfactant is present in an amount sufficient to attain critical micelle concentration of the compound of Formula I.

5. The pharmaceutically acceptable composition according to claim 1, further comprising a pharmaceutically acceptable aqueous liquid.

6. The pharmaceutically acceptable composition according to claim 1, wherein the compound having the structure according to Formula I is present in an amount of about 0.5 mg to about 100 mg.

7. The pharmaceutically acceptable composition according to claim 6, wherein the compound having the structure according to Formula I is present in an amount of about 25 mg to about 50 mg.

8. A compound of Formula 1: anionic surfactant ion pair wherein the compound of Formula I has the structure: 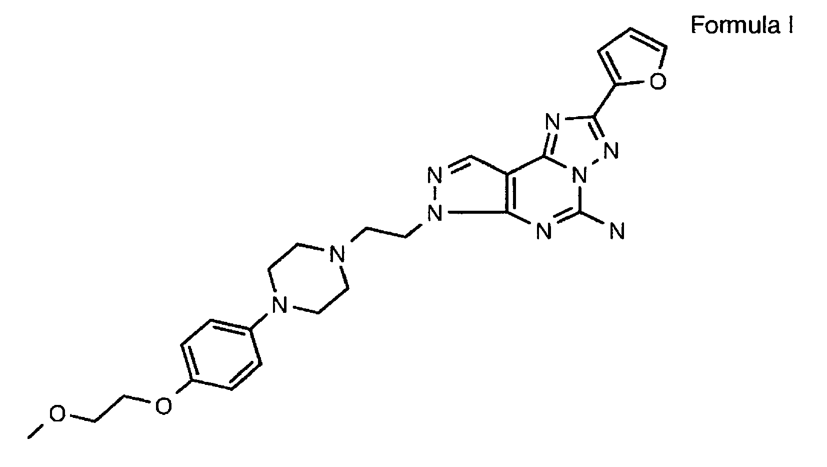 wherein the substituent shown as "N" represents NH₂, and the anionic surfactant is sodium docusate.

## Patentansprüche

1. Eine pharmazeutisch annehmbare Zusammensetzung, umfassend:
a) eine Verbindung mit der Struktur gemäß Formel I 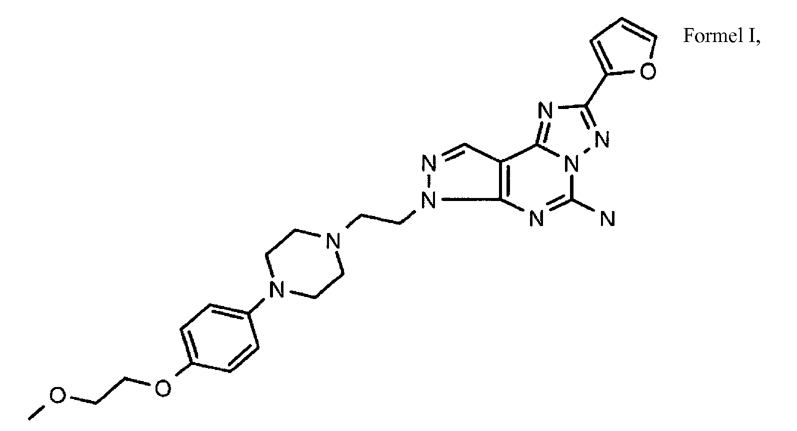 wobei der als "N" dargestellte Substituent NH₂ bedeutet,
b) wenigstens einen pharmazeutisch annehmbaren nichtwässrigen flüssigen Träger, wobei der flüssige Träger mit einem wässrigen Träger mischbar ist,
c) wenigstens ein Säuerungsmittel und
d) wenigstens ein anionisches Tensid, wobei das wenigstens eine anionische Tensid ein Ionenpaar mit der Verbindung der Formel I bildet und Natriumdocusat ist.

2. Die pharmazeutisch annehmbare Zusammensetzung gemäß Anspruch 1, die ferner wenigstens ein nichtionisches Tensid umfasst.

3. Die pharmazeutisch annehmbare Zusammensetzung gemäß Anspruch 2, wobei das nichtionische Tensid ausgewählt ist aus der Gruppe, bestehend aus Blockcopolymeren von Ethylenoxid und Propylenoxid, Glycol- oder Glycerinestern von gesättigten oder ungesättigten C₈- bis C₂₀-Säuren, Polyoxyethylenestern von gesättigten oder ungesättigten C₈- bis C₂₀-Säuren, Polyoxyethylenethern von gesättigten oder ungesättigten C₈- bis C₂₀-Säuren, Polyvinylalkoholen oder Sorbitestern von gesättigten oder ungesättigten C₁₀- bis C₂₀-Säuren.

4. Die pharmazeutisch annehmbare Zusammensetzung gemäß Anspruch 2, wobei das wenigstens eine nichtionische Tensid in einer Menge vorliegt, die ausreicht, um eine kritische Mizellkonzentration der Verbindung der Formel I zu erzielen.

5. Die pharmazeutisch annehmbare Zusammensetzung gemäß Anspruch 1, die ferner eine pharmazeutisch annehmbare wässrige Flüssigkeit umfasst.

6. Die pharmazeutisch annehmbare Zusammensetzung gemäß Anspruch 1, wobei die Verbindung mit der Struktur gemäß Formel I in einer Menge von etwa 0,5 mg bis etwa 100 mg vorliegt.

7. Die pharmazeutisch annehmbare Zusammensetzung gemäß Anspruch 6, wobei die Verbindung mit der Struktur gemäß Formel I in einer Menge von etwa 25 mg bis etwa 50 mg vorliegt.

8. Ein Verbindung der Formel I : anionisches Tensid-Ionenpaar, wobei die Verbindung der Formel I die Struktur 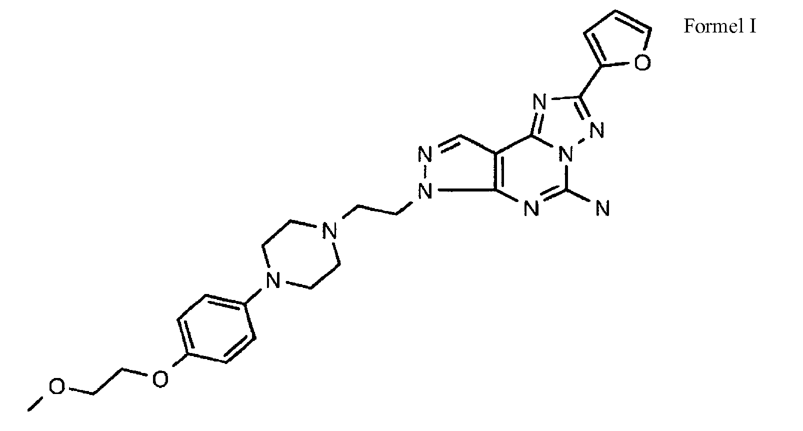 besitzt, wobei der als "N" dargestellte Substituent NH₂ bedeutet und das anionische Tensid Natriumdocusat ist.

## Revendications

1. Composition pharmaceutiquement acceptable comprenant:
a) un composé ayant la structure selon la Formule I 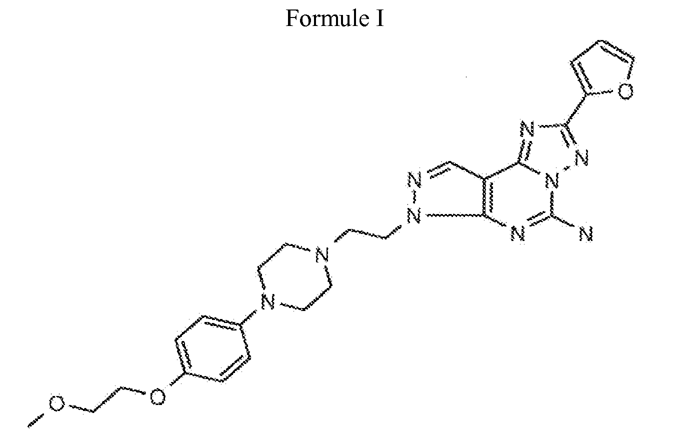 où le substituant représenté par "N" représente NH2;
b) au moins un véhicule liquide non aqueux pharmaceutiquement acceptable, où le véhicule liquide est miscible avec un véhicule aqueux;
c) au moins un agent acidifiant; et
d) au moins un tensioactif anionique, où ledit au moins un tensioactif anionique forme une paire d'ions avec le composé de la Formule I et est du docusate sodique.

2. Composition pharmaceutiquement acceptable selon la revendication 1, comprenant en outre au moins un tensioactif non ionique.

3. Composition pharmaceutiquement acceptable selon la revendication 2, dans laquelle le tensioactif non ionique est sélectionné parmi le groupe consistant en copolymères blocs d'oxyde d'éthylène et d'oxyde de propylène, en esters de glycol ou de glycéryle d'acides saturés ou insaturés en C8 à C20, en esters de polyoxyéthylène d'acides saturés ou insaturés en C8 à C20, en éthers de polyoxyéthylène d'acides saturés ou insaturés en C8 à C20, en alcools polyvinyliques ou en esters de sorbitan d'acides saturés ou insaturés en C10 à C20.

4. Composition pharmaceutiquement acceptable selon la revendication 2, dans laquelle le au moins un tensioactif non ionique est présent en une quantité suffisante pour atteindre une concentration critique de micelles du composé de la Formule I.

5. Composition pharmaceutiquement acceptable selon la revendication 1, comprenant en outre un liquide aqueux pharmaceutiquement acceptable.

6. Composition pharmaceutiquement acceptable selon la revendication 1, dans laquelle le composé ayant la structure selon la Formule I, est présent en une quantité d'environ 0,5 mg à environ 100 mg.

7. Composition pharmaceutiquement acceptable selon la revendication 6, dans laquelle le composé ayant la structure selon la Formule I est présent en une quantité d'environ 25 mg à environ 50 mg.

8. Paire d'ions du composé de la Formule I : tensioactif anionique, où le composé de la Formule I a la structure: 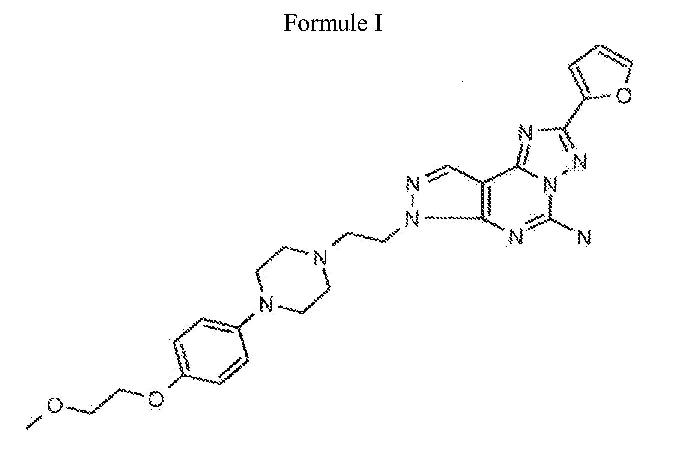 où le substituant représenté par "N" représente NH2, et le tensioactif anionique est du docusate sodique.
